# EUROPEAN PATENT APPLICATION

(11) **EP 4 781 913 A1**
(43) Date of publication of application: **29.07.2026**
(21) Application number: 25153271.9
(22) Date of filing: 22.01.2025
(51) Int. Cl.: A61B 6/06, A61B 6/00

(54) **DYNAMIC GEOMETRY ADAPTION DURING ROTATION OF AN X-RAY IMAGE**

(71) Applicant: Koninklijke Philips N.V., 5656 AE Eindhoven (NL)
(72) Inventor: BANERJEE, Amar Satyabroto, Eindhoven (NL)
(74) Representative: Philips Intellectual Property & Standards

(57) **Abstract**

The present invention relates to a computer-implemented method for dynamic geometry adaption during rotation of an X-ray image. The method comprises receiving a first X-ray image comprising a region of interest of a subject, receiving a plurality of second X-ray images of the subject, an imaging region of the plurality of second X-ray images being adjacent to and surrounding the region of interest of the subject, stitching the plurality of second X-ray images together, thereby generating a stitched image, and registering the first X-ray image onto the stitched image, thereby generating a combined image. The method comprises further receiving an input from a user defining a rotation angle, rotating the combined image by the rotation angle around a central point of the combined image, thereby generating a rotated combined image, determining an image section of the rotated combined image, wherein an orientation of the image section of the rotated combined image is the same as an orientation of the first X-ray image, and providing the determined image section of the rotated combined image.

## Description

### FIELD OF THE INVENTION

The present invention relates to a computer-implemented method for dynamic geometry adaption during rotation of an X-ray image, a data processing apparatus, an X-ray imaging system, a computer program, and a computer-readable storage medium.

### BACKGROUND OF THE INVENTION

Mobile C-arm X-ray machines are widely used in medical imaging for their ability to provide real-time visualization during various procedures. These machines may shape the X-ray beam using collimators, which can be square, hexagonal, circular, or may have any other shape. However, when these images are rotated, they often require cropping to keep the focus within the visible area. This cropping leads to images with cut corners or non-square shapes, reducing the amount of visible information and complicating the interpretation of the X-ray images.

Currently, radiologists face challenges in interpreting these cropped images, which can result in diagnostic errors. Moreover, the need for multiple shots to achieve a broader field of view after rotation of the image may increase radiation exposure, posing additional risks to patients. Thus, there is a clear need for a solution that maintains square images regardless of rotation of the image after acquisition, ensuring a complete and consistent visual field, thereby improving diagnostic reliability and reducing radiation exposure.

### SUMMARY OF THE INVENTION

It is an object of the present invention to provide an improved method that solves at least a part of the problems of the state of the art of how to maintain image consistency and image quality during rotation of X-ray images.

The inventors of the present invention have developed a method that provides a consistent and complete visual field when rotating the image, thereby significantly improving the reliability and accuracy of medical diagnoses.

The object of the present invention is solved by the subject matter of the independent claims, wherein further embodiments are incorporated in the dependent claims.

The described embodiments pertain to a computer-implemented method for dynamic geometry adaption during rotation of an X-ray image, a data processing apparatus, an X-ray imaging system, a computer program, and a computer-readable storage medium. The embodiments described further may be combined in any possible way. Synergistic effects may arise from different combinations in various ways of the embodiments described further although these combinations might not be described explicitly in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method, unless explicitly mentioned to the contrary hereinafter.

According to a first aspect of the invention, there is provided a computer-implemented method for dynamic geometry adaption during rotation of an X-ray image. The method comprises receiving a first X-ray image comprising a region of interest of a subject, receiving a plurality of second X-ray images of the subject, an imaging region of the plurality of second X-ray images being adjacent to and surrounding the region of interest of the subject, stitching the plurality of second X-ray images together, thereby generating a stitched image, and registering the first X-ray image onto the stitched image, thereby generating a combined image. The method comprises further receiving an input from a user defining a rotation angle, rotating the combined image by the rotation angle around a central point of the combined image, thereby generating a rotated combined image, determining an image section of the rotated combined image, wherein an orientation of the image section of the rotated combined image is the same as an orientation of the first X-ray image, and providing the determined image section of the rotated combined image.

Thus, the invention proposes an enhanced method and an imaging system that maintains square images regardless of rotation of the image. After identification of a target area and optimal positions and angles for image capture, a plurality of collimated images around the target area is captured in addition to the main image of the center region, preferably automatically. These images are dynamically stitched to create a high-resolution composite image of the center region comprising the region of interest and adjacent surrounding regions to ensure that any rotated image remains square by integrating relevant areas from the initial captures of the regions surrounding the center region. The images may be processed by an AI model that dynamically stitches and enhances the images. Laser-based geometry marker may be used to accurately mark and identify the target area and optimal angles for image capture.

As the current practice in mobile C-arm X-ray imaging faces significant challenges due to the collimator-shaped x-ray beams, which may lead to cropped images with cut corners or non-square shapes when rotated, the proposed method according to the invention solves these issues by introducing an enhanced imaging system and a method that maintains square images regardless of rotation of the image. The reduced visible information complicating image interpretation, and the increased risk of diagnostic errors is reduced by the method of the invention that may utilize an AI model and laser-based geometry marking to acquire and process images that provide a consistent field of view even when rotated.
Thus, the technical effect of the invention can be understood as creating high resolution composite images that remain square even during rotation by integrating collimated images captured initially with the help of a marker, e.g., a laser-based geometry marker.

Thus, by dynamic geometric adaptation, true square image can be provided even after image rotation.

In an embodiment of the invention, the first X-ray image is a square X-ray image, preferably a quadratic X-ray image, and an aspect ratio of the image section of the rotated combined image is equal to an aspect ratio of the first X-ray image. While the image, and in particular the content of the image, is rotated, the outline or the outer boarder of the image is not rotated. Thus, when displayed on a display unit, the image remains square, and the outline of the image does not change and does not rotate, however, the content of the image that is displayed is rotated, such that no image features can enter the visual display frame, in particular at the edges of the image.

In an embodiment of the invention, a size of the image section of the rotated combined image is equal to a size of the first X-ray image, or the size of the image section of the rotated combined image is different from the size of the first X-ray image.

Thus, the image can be cropped to an area that is either larger or smaller than the region of interest. As the combined image also contains the additional area apart from the region of interest, varying crop can be handled. In addition, the angle of rotation can also be applied accordingly.

In an embodiment of the invention, an image quality of the plurality of second X-ray images of the subject is lower than an image quality of the first X-ray image.

In an embodiment of the invention, the image section of the rotated combined image comprises a first portion and a second portion, wherein the first portion corresponds to a portion of the first X-ray image, and wherein the second portion corresponds to a portion of the plurality of second X-ray images.

In an embodiment of the invention, the method comprises further enhancing an image quality of at least a third portion of at least one of the plurality of second X-ray images, the stitched image, the combined image, the rotated combined image, and the image section of the rotated combined image.

In an embodiment of the invention, the at least third portion corresponds to the second portion of the image section of the rotated combined image.

In an embodiment of the invention, the plurality of second X-ray images comprises four second X-ray images, wherein each of the second X-ray images has a shape of an isosceles right-angled triangle, and wherein the second X-ray images are stitched together such that the generated stitched image comprises a shape having an outer contour of a first square and comprising an opening with a contour of a second square, the second square being rotated with respect to the first square by an angle of 45 degrees.

In an embodiment of the invention, when registering the first X-ray image onto the stitched image, the first X-ray image is registered onto the stitched image such that the opening of the stitched image is filled with the first X-ray image, and the size of the first X-ray image is equal to a size of the opening of the stitched image, or the size of the first X-ray image is larger than the size of the opening of the stitched image such that the first X-ray image and the stitched image overlap each other at least partially.

In an embodiment of the invention, the method comprises further receiving an input from a user defining the region of interest of the subject, and sending a request to an X-ray imaging system to acquire and provide the first X-ray image and the plurality of second X-ray images of the subject.

In an embodiment of the invention, the input of the user defining the region of interest of the subject is provided via a laser-based geometry marking system, and/or the X-ray imaging system comprises an automatically controlled image capture system, preferably a mobile c-arm system, comprising an automatically controlled collimation system.

In an embodiment of the invention, at least a part of the method is performed by a trained artificial intelligence.

Thus, the method of the invention may leverage multiple artificial intelligence AI models to enhance the functionality and accuracy of mobile C-arm X-ray imaging. A convolutional neural network CNN can be primarily employed for image stitching and enhancement due to its robust feature extraction capabilities. The CNN can be trained on a diverse dataset of annotated X-ray images, ensuring it can accurately map and align features from different angles. Additionally, a probabilistic graphical model can be utilized for feature mapping and stitching, providing a dynamic approach to integrating multiple images into a single, coherent composite image. An adaptive oriented registration algorithm may align the region of interest with the stitched images, accounting for varying orientations and ensuring precise placement. Finally, an oriented view-matching fusion technique can combine the enhanced areas with the region of interest using orientation and view data to ensure seamless integration. These models work together to maintain square images regardless of rotation, improve image quality, and enhance diagnostic accuracy. The AI model can be developed using machine learning algorithms, trained on a dataset of X-ray images. It may include modules for probabilistic feature mapping, adaptive oriented registration, low-dose enhancement, and oriented view-matching fusion.

In an embodiment of the invention, the method comprises further providing the determined image section of the rotated combined image to a display unit.

According to another aspect of the invention, there is provided a data processing apparatus for carrying out the steps of the method according to any one of the preceding embodiments.

According to another aspect of the invention, there is provided an X-ray imaging system comprising the data processing apparatus according to the preceding embodiment, an automatically controlled image capture system, a user input device, and a display unit.

According to another aspect of the invention, there is provided a computer program comprising instructions which, when the program is executed by a computer, cause the computer to carry out the steps of the method according to any one of the preceding embodiments.

According to another aspect of the invention, there is provided a computer-readable storage medium comprising instructions which, when executed by a computer, cause the computer to carry out the steps of the method according to any one of the preceding embodiments.

Thus, the benefits provided by any of the above aspects equally apply to all of the other aspects and vice versa.

In summary, the invention relates to a computer-implemented method for dynamic geometry adaption during rotation of an X-ray image. The method comprises receiving a first X-ray image comprising a region of interest of a subject, receiving a plurality of second X-ray images of the subject, an imaging region of the plurality of second X-ray images being adjacent to and surrounding the region of interest of the subject, stitching the plurality of second X-ray images together, thereby generating a stitched image, and registering the first X-ray image onto the stitched image, thereby generating a combined image. The method comprises further receiving an input from a user defining a rotation angle, rotating the combined image by the rotation angle around a central point of the combined image, thereby generating a rotated combined image, determining an image section of the rotated combined image, wherein an orientation of the image section of the rotated combined image is the same as an orientation of the first X-ray image, and providing the determined image section of the rotated combined image.

One of the advantages of embodiments of the present invention is that the creation of high-resolution composite images is ensured that remain square and consistent regardless of rotation of the image. Another advantage may reside in that overall efficiency of medical imaging processes may be improved by enhancing the reliability and usability of mobile C-arm X-ray machines. The practical challenges faced by radiologists may be at least partially solved by the proposed method of the invention. Further, radiation exposure and human error may be reduced. The AI-based image stitching and enhancement could be integrated as a new software feature within existing medical imaging software platforms, enhancing their capabilities.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a block diagram of a computer-implemented method for dynamic geometry adaption during rotation of an X-ray image according to an embodiment of the invention.
Fig. 2A shows a schematic illustration of the arrangement of the plurality of second X-ray images.
Fig. 2B shows a schematic illustration of the first X-ray image stitched to the plurality of second X-ray images.
Fig. 2C shows a schematic illustration of the rotated combined image and the determined image section of the rotated combined image.
Fig. 3 shows a schematic setup of an X-ray imaging system according to an embodiment of the invention acquiring the plurality of second X-ray images.

### DETAILED DESCRIPTION OF EMBODIMENTS

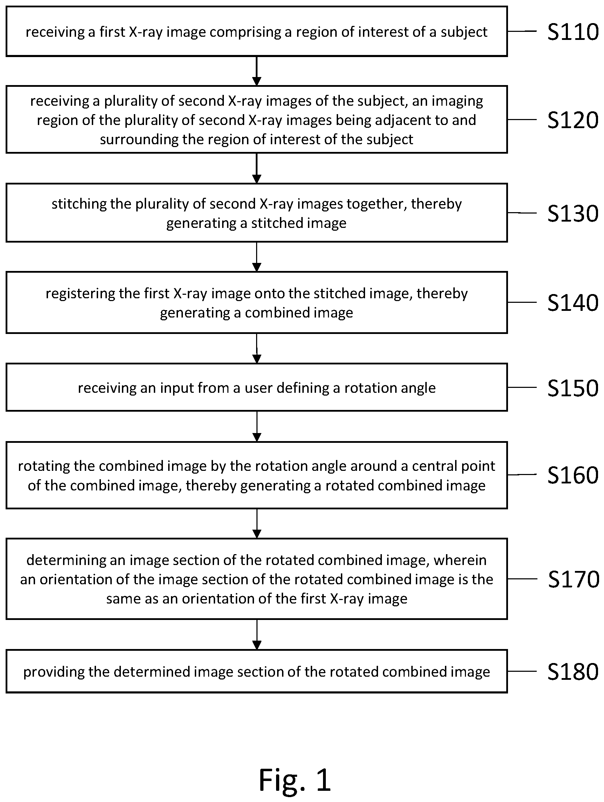

Fig. 1 shows a block diagram of a computer-implemented method for dynamic geometry adaption during rotation of an X-ray image according to an embodiment of the invention. The method comprises step S110 of receiving a first X-ray image 110 comprising a region of interest 130 of a subject 100, and step S120 of receiving a plurality of second X-ray images 120 of the subject 100, an imaging region of the plurality of second X-ray images 120 being adjacent to and surrounding the region of interest 130 of the subject 100. The method comprises further step S130 of stitching the plurality of second X-ray images 120 together, thereby generating a stitched image, and step S140 of registering the first X-ray image 110 onto the stitched image, thereby generating a combined image. The method comprises further step S 150 of receiving an input from a user defining a rotation angle α, and step S160 of rotating the combined image by the rotation angle α around a central point of the combined image, thereby generating a rotated combined image 140. Further, the method comprises step S170 of determining an image section 150 of the rotated combined image, wherein an orientation of the image section 150 of the rotated combined image is the same as an orientation of the first X-ray image 110, and step S180 of providing the determined image section 150 of the rotated combined image.

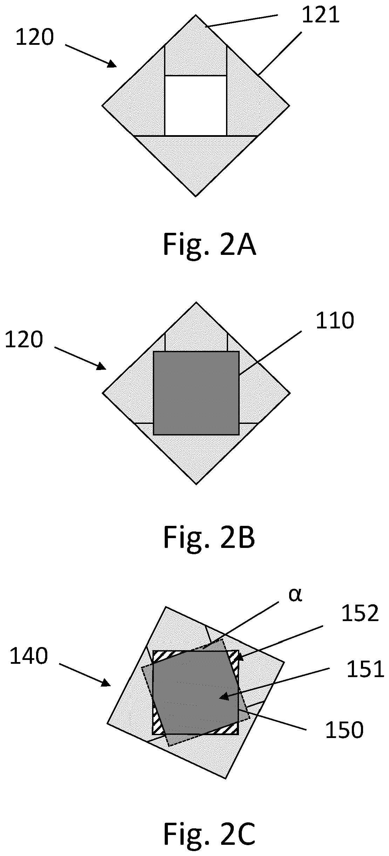

Fig. 2A shows a schematic illustration of the arrangement of the plurality of second X-ray images 120. In this embodiment of the invention, the plurality of second X-ray images 120 comprises four second X-ray images 121, each of which has a shape of an isosceles right-angled triangle, and the second X-ray images are stitched together such that the generated stitched image comprises a shape having an outer contour of a first square and comprising an opening with a contour of a second square, the second square being rotated with respect to the first square by an angle of 45 degrees.

Thus, when registering the first X-ray image 110 onto the stitched image, the first X-ray image 110 is registered onto the stitched image such that the opening of the stitched image is filled with the first X-ray image, and the size of the first X-ray image is equal to a size of the opening of the stitched image, or the size of the first X-ray image is larger than the size of the opening of the stitched image such that the first X-ray image and the stitched image overlap each other at least partially. As is shown in Fig. 2B, the first X-ray image 110 fills the opening of the stitched image comprised of the plurality of second X-ray images 120, and slightly overlaps with the plurality of second X-ray images 120. By registering the first X-ray image 110 to the stitched image, a combined image is generated. As an image quality of the plurality of second X-ray images 120 of the subject 100 may be lower than an image quality of the first X-ray image 110, optionally, an image quality of at least a third portion of at least one of the plurality of second X-ray images 120, the stitched image, the combined image, the rotated combined image 140, and the image section 150 of the rotated combined image can be enhanced. Preferably, the at least third portion corresponds to the second portion 152 of the image section 150 of the rotated combined image.

Fig. 2C shows a schematic illustration of the rotated combined image 140 and the determined image section 150 of the rotated combined image. The combined image is rotated according to a user input by a predefined angle α to generate the rotated combined image 140 as shown in Fig. 2C. An image section 150 of the rotated combined image is determined, an orientation of the image section 150 being the same as an orientation of the first X-ray image 110. In the example shown in Fig. 2A to 2C, both the first X-ray image 110 and the image section 150 are square images, preferably quadratic images, and an aspect ratio of the image section of the rotated combined image is equal to an aspect ratio of the first X-ray image. Therefore, the first X-ray image 110 and the image section 150 are both oriented such that they are aligned parallel and orthogonal to the respective borders of a frame of a display unit, i.e., they are horizontally and vertically aligned. Thus, while the image, and in particular the content of the image, is rotated, the outline or the outer boarder of the image is not rotated. Thus, when displayed on a display unit, the image remains square, and the outline of the image does not change and does not rotate. A size of the image section 150 of the rotated combined image may be equal to a size of the first X-ray image 110 as shown in Fig. 2C, or the size of the image section of the rotated combined image may be different from the size of the first X-ray image. Therefore, in this example, the image section 150 of the rotated combined image comprises a first portion 151 and a second portion 152, wherein the first portion corresponds to a portion of the first X-ray image 110, and the second portion 152 corresponds to a portion of the plurality of second X-ray images 120. As shown in Fig 2C, the second portion 152 is illustrated as hatched area, which is added to the first portion 152 of the image section 150 that is derived from the first X-ray image 110. In this example, the first portion 151 and the second portion 152 add up to the square image section 150, which can be provided to a display unit. Preferably, at least a part of the above-described method is performed by a trained artificial intelligence.

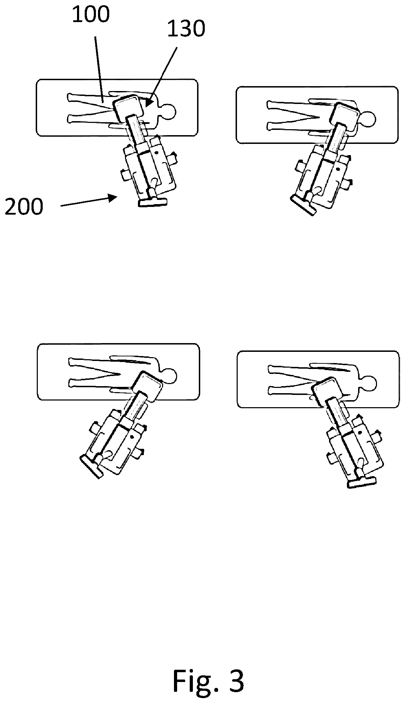

Fig. 3 shows a schematic setup of an X-ray imaging system 200 according to an embodiment of the invention acquiring the plurality of second X-ray images 120. An input of a user may define a region of interest 130 of a subject 100, preferably via a laser-based geometry marking system. The input from the user defining the region of interest of the subject is then received from the marking system, and a request can be sent to the X-ray imaging system 200 to acquire and provide the first X-ray image 110 and the plurality of second X-ray images 120 of the subject 100. The X-ray imaging system 200 can comprise an automatically controlled image capture system, preferably a mobile c-arm system, an optionally an automatically controlled collimation system. As shown in Fig. 3, the X-ray imaging system 200 acquires four second X-ray images 121 of an area surrounding the defined region of interest 130 of the subject 100 via an automated movement of the arm to take suitable collimated images of the identified region. Actual image data of the region of interest taken during the procedure can now be stitched and fused with the four images to create a large area image. After rotating the actual live image, the model identifies the image parts that need to be taken from the large area image in addition to the first X-ray image.

Thus, an AI-based system and methodology for intra-operative true-square imaging while rotation on the live image from an X-ray machine is provided. As explained, mobile C-arm X-ray machines, that are widely used in medical imaging, face significant challenges and disadvantages with current methods and devices. When x-ray images are rotated, they often need to be cropped to maintain focus within the visible area. This cropping results in images with cut corners or non-square shapes, such as circles or hexagons, which reduces the amount of visible information and may lead to a loss of information. This loss of information complicates image interpretation, increases the risk of diagnostic errors, and can lead to missed or incorrect diagnoses. To achieve a broader view after rotating the image, multiple shots are often required, leading to increased radiation exposure for patients, which poses additional health risks to patients, which is a significant concern in medical imaging. Further, the current process often requires manual intervention to select and rotate images, which can lead to inconsistencies and human errors. Manual processes are time-consuming and increase the workload on medical staff, potentially compromising the efficiency and accuracy of imaging procedures.

The technical and clinical challenges of maintaining image consistency and quality during rotation are not adequately addressed by current collimator-based imaging methods as known in the art. Inconsistent image quality during rotation can hinder accurate diagnosis and treatment planning. Clinically, the limitations of current imaging methods lead to increased errors and unreliable X-ray images. In case the cropped part of the image is to be seen, another X-ray shot is required after moving the C-arm to the desired position, consuming additional time and radiation. These issues can negatively impact patient outcomes and the overall effectiveness of medical imaging procedures.

Thus, the present invention solves these problems with true square imagining. The need to rotate and re-position the C-arm again to get the broader view is reduced, as well as the radiation to take multiple shots after repositioning. A full square image of the area can be provided to the surgeon after rotating the image on the user interface or on a display unit. It can be ensured that the whole target anatomy is covered on the screen at all times without any unwanted cropping.

Therefore, a primary purpose of this invention is to improve ease of usage and visualization of the image during the surgery. The invention is targeted to reduce the radiation while making multiple pictures for various re-adjustments of the C-arm.

The system can utilize a laser-based geometry marker to accurately identify the target area and optimal angles for image capture. It then automatically captures four collimated images around the target area, minimizing radiation exposure and human error. An AI model dynamically stitches these images to create a high-resolution composite image and ensures that any rotated image remains square by integrating relevant areas from the initial captures. This method provides a consistent and complete visual field, significantly improving the reliability and accuracy of medical diagnoses.

To build the system, a laser-based geometry marker can be installed on the C-arm, ensuring it is aligned with the X-ray source. The laser module can be connected to the control software, allowing the operator to activate and adjust the laser for precise target area marking. The C-arm can be equipped with motorized actuators and sensors for precise positioning, to develop an automated image capture system. The control software needs to be developed to automate the capture of four collimated images based on the laser marker's guidance. To develop an AI-based image stitching and enhancement model using machine learning techniques, it can be trained on a dataset of X-ray images. The AI model can be integrated into the imaging software, enabling it to stitch and enhance images dynamically. Real-time processing capabilities can be embedded into the AI model, allowing it to manage image rotation and adjustment dynamically. Thus, it is assured that the AI model can adjust and integrate relevant areas from the initially captured images to maintain square dimensions during rotation.

Thus, the system according to the invention can comprise several technical features that collectively solve the problems associated with current mobile C-arm X-ray imaging methods. These features ensure improved image quality, reduced radiation exposure, and minimized human error. Below are technical elements of the invention summarized.

A laser-based geometry marker can be used to precisely identify and mark the target area for imaging. This marker can be manually operated to mark the area of interest of the patient. This marker ensures accurate localization of the target area, guiding the C-arm to find optimal angles for image capture, and thus can reduce the risk of incorrect positioning and ensure comprehensive coverage of the target area.

An automated image capture system may capture for example four collimated images around the target area. The system may capture images from different angles with minimal radiation dose, reducing the need for manual intervention. Based on the region of interest marked with the laser markers, the system can control a motorized C-arm to reach to suitable positions to take the initial images. These initial images can be taken with a triangular collimated geometry and with low dose in order to minimize the dose. This minimizes radiation exposure, reduces human error, and ensures consistent image quality.

Auto-collimation or automatic setting of the collimator plates allows to match the capturing area calculated for each position. The model identifies the collimation required to take the image in the various above positions. This is done to reduce the radiation and still cover optimal area for the imaging.

AI-based image stitching and enhancement dynamically stitches and enhances images. The model registers the region of interest within the four captured images. The AI model can perform several key tasks. Probabilistic feature mapping stitches the initial four collimated images to create a larger area image. Adaptive oriented registration allows to register the high-dose region of interest onto the larger stitched image. Low-dose enhancement can enhance the identified additional areas from the low-dose images to match the quality of the region of interest. Oriented view-matching fusion can integrate the enhanced areas with the region of interest image seamlessly. Thus, the creation of high-resolution composite images that remain square and consistent regardless of rotation can be ensured.

Real-time image rotation and adjustment by the AI model adjusts and integrates relevant areas from the initially captured images to produce a rotated version that remains square, and thus maintains a consistent visual field, facilitating accurate diagnosis and reducing the need for multiple images. A consistent square shape of the images, without cut corners or loss of information is provided.

The proposed embodiments of the invention can be applied in various technical and commercial fields, particularly within the domain of medical imaging. Key areas of application can include mobile C-arm X-ray machines with enhanced functionality of by ensuring square images regardless of rotation, improving image quality and diagnostic accuracy. This feature is particularly beneficial for intraoperative imaging, providing surgeons with consistent and reliable images during procedures. Further, the methods and systems of the invention can be integrated into fixed X-ray systems used in radiology departments, providing consistent and square imaging across various procedures. These systems may benefit from the enhanced imaging quality and reduced radiation exposure, making them more attractive to healthcare providers. Further, the principles of true square rotation and automated image capture can be adapted for use in intraoperative CT systems, ensuring high-resolution, consistent images during surgical procedures. The respective features to provide real-time, accurate imaging, enhancing surgical precision and patient outcomes can be integrated.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

It is to be understood that any of the previous steps described in relation to embodiments and/or training steps described above can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. In embodiments, a computer system performs logical processing based on digital signals received via an analogue-to-digital converter.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage devices. Portions of the present disclosure include processes and instructions that may be embodied in software, firmware, or hardware, and when embodied in software, may be downloaded to reside on and be operated from different platforms used by a variety of operating systems.

In a networked deployment, the computer system operates in the capacity of a server, or as a client user computer in a server-client user network environment, or as a peer computer system in a peer-to-peer or distributed network environment. The computer system can also be implemented as or incorporated into various devices, such as a server or another type of computer such as a workstation that includes a controller, a stationary computer, a mobile computer, a personal computer (PC), a laptop computer, a tablet computer, or any other machine capable of executing a set of software instructions sequentially or non-sequentially that specify actions to be taken by that machine. The computer system can be incorporated as an integrated system part of a larger system that includes additional devices. In an embodiment, the computer system can be implemented using electronic devices that provide voice, video, or data communication possibilities. Further, while the computer system is illustrated in the singular, the term "system" shall also be taken to include any collection of systems or sub-systems that individually or jointly execute a set or multiple sets, of software instructions to perform one or more computer functions.

The computer system may also include a processor. The processor executes instructions to implement some, or all aspects of methods and processes described herein. The processor is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor is an article of manufacture and/or a machine component. The processor is configured to execute software instructions to perform functions as described in the various embodiments herein. The processor may be a general-purpose processor or may be part of an application specific integrated circuit (ASIC). The processor may also be a microprocessor, a microcomputer, a processor chip, a controller, a microcontroller, a digital signal processor (DSP), a state machine, or a programmable logic device, a logical circuit, including a programmable gate array (PGA), such as a field programmable gate array (FPGA), or another type of circuit that includes discrete gate and/or transistor logic. The processor may be a central processing unit (CPU), a graphics processing unit (GPU), or both. Additionally, any processor described herein may include multiple processors, parallel processors, or both. Multiple processors may be included in, or coupled to, a single device or multiple devices. The processor can include one or more internal levels of cache, and a bus controller or bus interface unit to direct interaction with a bus. The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection, or network, of computing devices each including a processor or processors. Programs have software instructions performed by one or multiple processors that may be within the same computing device or which may be distributed across multiple computing devices. Further, the software instructions, when executed by the processor, perform one or more steps of the methods and processes as described herein.

The computer system can further include a communications interface by way of which the computer system can connect to networks and receive data useful in executing the methods and system set out herein as well as transmitting information to other devices. The computer system further includes a video display unit as an output device by which information can be output, such as a liquid crystal display (LCD), an organic light emitting diode (OLED), a flat panel display, a solid-state display, or a cathode ray tube (CRT), for example. Additionally, the computer system includes an input device, such as a keyboard/virtual keyboard or touch-sensitive input screen or speech input with speech recognition, and a cursor control device, such as a mouse or touch-sensitive input screen or pad. The computer system also optionally includes a disk drive unit, a signal generation device, such as a speaker or remote control, and/or a network interface device.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any programming language that is sufficient for achieving the techniques and implementations of the present disclosure may be used. In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the Figures are to be regarded as illustrative rather than restrictive.

### LIST OF REFERENCE SIGNS:

- 100: subject
- 110: first X-ray image
- 120: plurality of second X-ray images
- 121: second X-ray image
- 130: region of interest
- 140: rotated combined image
- 150: image section
- 151: first portion
- 152: second portion
- 200: X-ray imaging system

## Claims

1. A computer-implemented method for dynamic geometry adaption during rotation of an X-ray image, the method comprising:
receiving (S110) a first X-ray image (110) comprising a region of interest (130) of a subject (100);
receiving (S120) a plurality of second X-ray images (120) of the subject (100), an imaging region of the plurality of second X-ray images (120) being adjacent to and surrounding the region of interest (130) of the subject (100);
stitching (S130) the plurality of second X-ray images (120) together, thereby generating a stitched image;
registering (S140) the first X-ray image (110) onto the stitched image, thereby generating a combined image;
receiving (S150) an input from a user defining a rotation angle (α);
rotating (S160) the combined image by the rotation angle (α) around a central point of the combined image, thereby generating a rotated combined image (140);
determining (S170) an image section (150) of the rotated combined image, wherein an orientation of the image section (150) of the rotated combined image is the same as an orientation of the first X-ray image (110); and
providing (S180) the determined image section (150) of the rotated combined image.

2. The method according to claim 1, wherein an image quality of the plurality of second X-ray images (120) of the subject (100) is lower than an image quality of the first X-ray image (110).

3. The method according to any one of the preceding claims, wherein the image section (150) of the rotated combined image comprises a first portion (151) and a second portion (152),
wherein the first portion (151) corresponds to a portion of the first X-ray image (110), and wherein the second portion (152) corresponds to a portion of the plurality of second X-ray images (120.

4. The method according to claim 3, comprising enhancing an image quality of at least a third portion of at least one of
the plurality of second X-ray images (120),
the stitched image,
the combined image,
the rotated combined image (140), and
the image section (150) of the rotated combined image.

5. The method according to claim 4, wherein the at least third portion corresponds to the second portion (152) of the image section (150) of the rotated combined image.

6. The method according to any one of the preceding claims, wherein the plurality of second X-ray images (120) comprises four second X-ray images (121), wherein each of the second X-ray images (121) has a shape of an isosceles right-angled triangle, and wherein the second X-ray images (121) are stitched together such that the generated stitched image comprises a shape having an outer contour of a first square and comprising an opening with a contour of a second square, the second square being rotated with respect to the first square by an angle of 45 degrees.

7. The method according to claim 6, wherein, when registering the first X-ray image (110) onto the stitched image, the first X-ray image is registered onto the stitched image such that the opening of the stitched image is filled with the first X-ray image, and
wherein the size of the first X-ray image (110) is equal to a size of the opening of the stitched image, or wherein the size of the first X-ray image (110) is larger than the size of the opening of the stitched image such that the first X-ray image and the stitched image overlap each other at least partially.

8. The method according to any one of the preceding claims, comprising
receiving an input from a user defining the region of interest of the subject (130), and
sending a request to an X-ray imaging system (200) to acquire and provide the first X-ray image (110) and the plurality of second X-ray images (120) of the subject (100).

9. The method according to claim 8, wherein the input of the user defining the region of interest (130) of the subject (100) is provided via a laser-based geometry marking system, and/or wherein the X-ray imaging system (200) comprises an automatically controlled image capture system, preferably a mobile c-arm system, comprising an automatically controlled collimation system.

10. The method according to any one of the preceding claims, wherein at least a part of the method is performed by a trained artificial intelligence.

11. The method according to any one of the preceding claims, comprising providing the determined image section (150) of the rotated combined image to a display unit.

12. A data processing apparatus for carrying out the steps of the method according to any one of claims 1 to 11.

13. An X-ray imaging system (200) comprising the data processing apparatus according to claim 12, an automatically controlled image capture system, a user input device, and a display unit.

14. A computer program comprising instructions which, when the program is executed by a computer, cause the computer to carry out the steps of the method according to any one of claims 1 to 11.

15. A computer-readable storage medium comprising instructions which, when executed by a computer, cause the computer to carry out the steps of the method according to any of claims 1 to 11.
